(19) **Europäisches Patentamt / European Patent Office / Office européen des brevets**

(11) **EP 4 559 497 A2**

(12) **EUROPEAN PATENT APPLICATION**

(43) Date of publication:
28.05.2025 Bulletin 2025/22

(21) Application number: 25162875.6

(22) Date of filing: 21.09.2021

(51) International Patent Classification (IPC):
**A61M 13/00** (2006.01)

(52) Cooperative Patent Classification (CPC):
**A61M 15/009; A61M 13/003;** A61M 11/02;
A61M 15/0005; A61M 15/0036; A61M 15/0041;
A61M 2202/0007; A61M 2202/0225;
A61M 2202/064; A61M 2205/8225; B05B 9/0833;
B05B 12/087

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 22.09.2020 US 202063081659 P

(62) Document number(s) of the earlier application(s) in
accordance with Art. 76 EPC:
**21790754.2 / 4 171 690**

(71) Applicant: **Boston Scientific Scimed, Inc.
Maple Grove, MN 55311 (US)**

(72) Inventors:
• **PIC, Andrew
Northboro, MA 01532 (US)**
• **EVERS, Ryan
Billerica, MA 01821 (US)**
• **MURRAY, Collin
Bolton, MA 01740 (US)**
• **CONGDON, Daniel
Hudson, MA 01749 (US)**
• **JAGELSKI, Matthew R.
Milford, MA 01757 (US)**

(74) Representative: **Vossius & Partner
Patentanwälte Rechtsanwälte mbB
Siebertstrasse 3
81675 München (DE)**

Remarks:
This application was filed on 11-03-2025 as a
divisional application to the application mentioned
under INID code 62.

(54) **PRESSURE RELEASE FOR MEDICAL DEVICES**

(57) A medical device configured to deliver an agent. The medical device comprises a body, a handle (30), a member (36), and an inlet (76).

The handle (30) defines a lumen (34), wherein the lumen (34) is configured to receive a container (50) containing the pressurized fluid. The member (36) is disposed at a proximal end of the handle (30), wherein the member (36) is configured to urge the container (50) within the handle (30) towards a distal direction into fluid communication with the body. The inlet (76) disposed at a distal end of the handle (30), wherein the inlet (76) includes a pierce pin configured to release the pressurized fluid from the container (50) in response to the member (36) urging the container (50) into the pierce pin.

FIG. 4

EP 4 559 497 A2

**Description**

**CROSS-REFERENCE TO RELATED APPLICATIONS**

This application claims the benefit of priority of U.S. Provisional Patent Application No. 63/081,659, filed September 22, 2020, the entirety of which is incorporated herein by reference.

**TECHNICAL FIELD**

The disclosure relates generally to medical systems and devices for delivering pressurized fluids, and in examples, to methods and tools for releasing fluid from a medical device that includes or is connected to a pressurized fluid source.

**BACKGROUND**

Fluid delivery systems and devices are used to supply various fluids, such as a gas, during medical procedures. These procedures may include supplying fluids within a range of appropriate pressures and/or flow rates. These fluids may include agents, e.g., hemostatic agents, optimally delivered to tissue at an appropriate pressure and/or flow rate, for the particular application.

Handheld medical fluid delivery systems often require delivering a fluid from a high pressure storage tank, such as a cartridge or similar housing. Such cartridges may be loaded into a handle of the delivery system and may energize or charge the delivery system when a seal on the cartridge is pierced by a pierce pin or similar device on the delivery system. These fluids may be under high pressures and may cause injury if the medical system is not used properly. In some instances, pressurized fluid may leak from the cartridges or containment devices into a sealed lumen of the handle of the medical fluid delivery systems. Over pressurization of the lumen and the handle may result in failure of the medical fluid delivery system and may result in injury to the user or the patient. The disclosure may solve one or more of these problems or other problems in the art. The scope of the disclosure, however, is defined by the attached claims and not the ability to solve a specific problem.

**SUMMARY OF THE DISCLOSURE**

According to an aspect, a device configured to deliver a pressurized fluid includes a body having an input opening for receiving the pressurized fluid and an output opening for delivering the pressurized fluid, a handle defining a lumen, wherein the lumen is configured to receive a container containing the pressurized fluid, and at least one aperture fluidly connecting the lumen to an atmosphere external of the device.

The at least one aperture may be defined by a wall of the handle.

The at least one aperture may include a plurality of apertures, and wherein the plurality of apertures may be arranged in an asymmetrical pattern on the handle.

A distal end of the handle may be configured to be connected to the body via screw threads, and wherein the at least one aperture may extend through a portion of the screw threads.

The handle may include one or more screw threads, wherein the body may include one or more screw threads, wherein the one or more apertures may be configured to be formed through the one or more screw threads of each of the handle and the body, and wherein the one or more apertures on the handle and the one or more apertures on the body may be configured to align when the handle is attached to the body.

The one or more apertures may be a single aperture disposed at a proximalmost end of the handle, and wherein the single aperture may face a proximal direction.

The device may include a plurality of crenellations and a plurality of openings defined between adjacent crenellations, and wherein the openings may be fluidly connected to the single aperture.

The plurality of crenellations may be configured to contact a proximalmost end of the container when the container is attached to the device.

A sum of a cross-sectional area of each of the plurality of openings may be equal to a cross-sectional area of the single aperture.

Only proximally facing surfaces of an outermost surface of the container may contact an innermost surface of the handle when the container is connected to the device.

The at least one aperture may be configured to release the pressurized gas in a time equal to or less than 0.5 seconds.

The body may be configured to form a hole in the container, wherein the pressurized fluid may be configured to pass from the container via the hole, and wherein a diameter of the hole may be less than or equal to 0.060 inches.

A cross-sectional area of a sum of each of the at least one aperture may be equal to or greater than approximately 0.25 square inches.

The device may further include the container, and a membrane covering each aperture of the at least one aperture, wherein the membrane may be configured to rupture when a pressure of the pressurized fluid within the lumen exceeds a threshold.

The pressurized fluid may have a pressure of approximately 14 pounds per square inch.

According to another aspect, a device configured to deliver a pressurized fluid includes a body having an input opening for receiving the pressurized fluid and an output opening for delivering the pressurized fluid, a handle defining a lumen, wherein the lumen is configured to receive a container containing the pressurized fluid, and a plurality of apertures fluidly connecting the lumen and an outer surface of the device, wherein a cross-sectional area of a sum of each of the plurality of apertures is equal to or greater than approximately 0.25 square inches, and wherein the pressurized fluid is configured to be released from the lumen in approximately 0.5 seconds or less.

The plurality of apertures may be arranged in an asymmetrical shape on the handle.

The handle may be connected to the body via a threaded connection, and wherein the plurality of apertures may pass through the threaded connection.

The device may further include a membrane covering each aperture of the at least one aperture, wherein the membrane may be configured to rupture when a pressure of the pressurized fluid within the lumen is equal to or greater than a threshold.

According to another aspect, a method for controlling a fluid delivery to a body of a patient includes inserting a container containing a pressurized fluid into a lumen of a handle of a fluid delivery device, the fluid delivery device defining one or more apertures fluidly connecting the lumen to an atmosphere external the fluid delivery device, and causing an opening in the container.

## BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.

FIG. 1 is a schematic of a delivery system according to an exemplary embodiment;
FIG. 2 is a side view of a handle of the delivery system of FIG. 1 according to an embodiment;
FIG. 3 is a cross-section of the handle of FIG. 2 according to an embodiment;
FIG. 4 is a cut-out view of a handle of the delivery system of FIG. 1 according to another embodiment;
FIG. 5A is a cross-section of a handle of the delivery system of FIG. 1 according to another embodiment; and
FIG. 5B is a cross-section of the handle of FIG. 5A according to an embodiment.

## DETAILED DESCRIPTION

The disclosure is described with reference to exemplary medical systems for dispensing an agent (such as a hemostatic agent) using a pressurized fluid. The devices associated with the medical systems may improve the functionality and/or the safety of the medical systems by venting pressurized fluids to atmosphere (e.g., air outside a handle and/or the medical system) if fluid from a pressurized fluid container leaks into an undesired location in the medical system. In examples, ventilation openings provided on or in the medical system may vent pressurized fluid from the lumen of the handle and may prevent over pressurization of the handle and/or the medical fluid delivery system.

Reference to any particular procedure is provided in this disclosure only for convenience and not intended to limit the disclosure. A person of ordinary skill in the art would recognize that the concepts underlying the disclosed device and application method may be utilized in any suitable procedure, medical or otherwise. The disclosure may be understood with reference to the following description and the appended drawings, wherein like elements are referred to with the same reference numerals.

Both the foregoing general description and the following detailed description are exemplary and explanatory only and are not restrictive of the features, as claimed. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus.

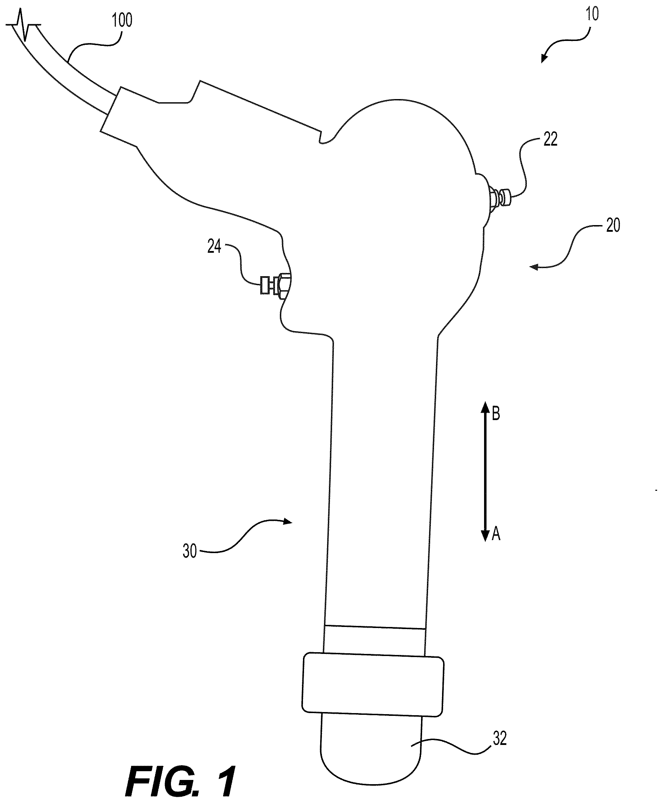

For ease of description, portions of the device and/or its components are referred to as proximal and distal portions. It should be noted that the term "proximal" is intended to refer to portions closer to a user of the device or upstream in a propellant fluid path (in the direction of arrow A in FIG. 1), and the term "distal" is used herein to refer to portions further away from the user or downstream in the propellant fluid path (in the direction of arrow B in FIG. 1). Similarly, extends "distally" indicates that a component extends in a distal direction, and extends "proximally" indicates that a component extends in a proximal direction. Further, as used herein, the terms "about," "approximately" and "substantially" indicate a range of values within +/- 10% of a stated or implied value. Additionally, terms that indicate the geometric shape of a component/surface refer only to approximate shapes.

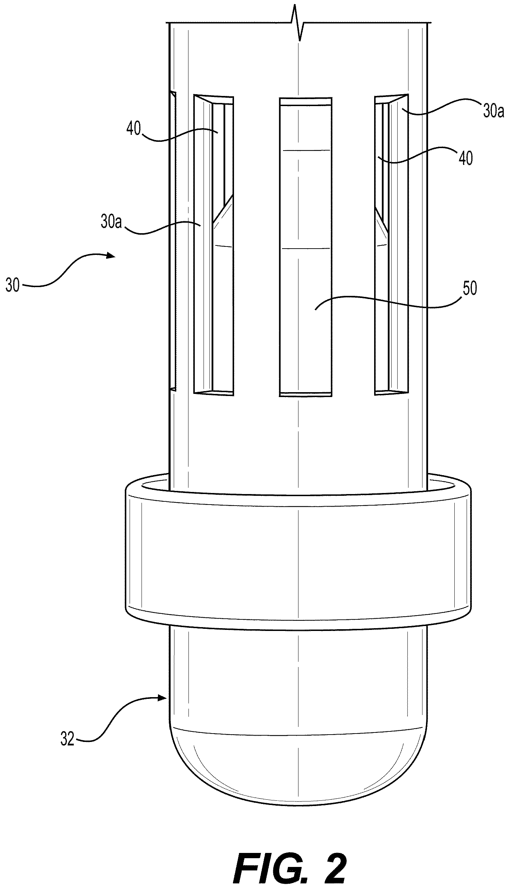

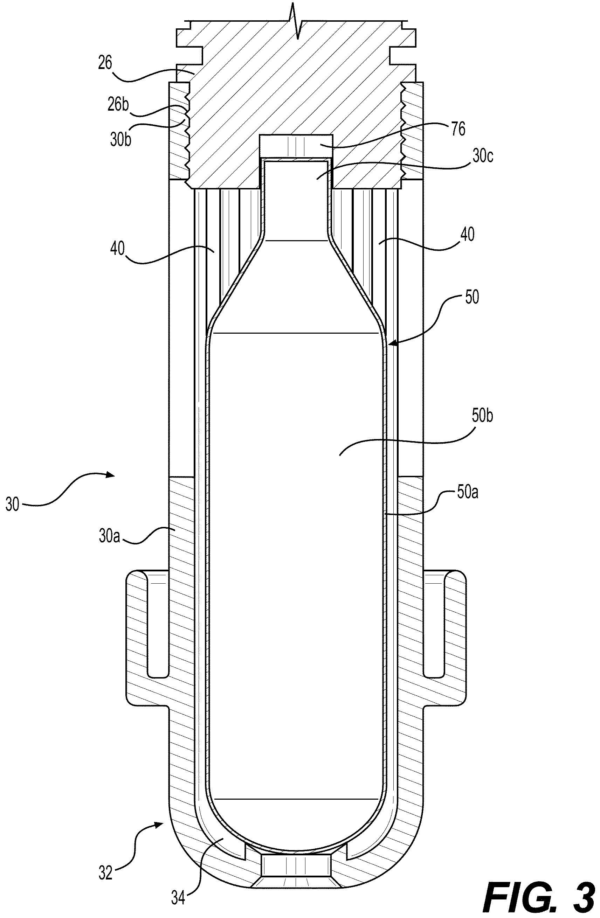

Referring to FIG. 1, a delivery system 10 according to an embodiment is shown. Delivery system 10 includes an application device 20, e.g., a hand-held device, having a handle 30 at a proximal end, and one or more triggers or actuators 22, 24 configured to actuate delivery system 10 to release a propellant fluid. A tube 100 (e.g., a catheter), or an application tip, may be attached to a distal outlet of delivery system 10 to aid in supplying the propellant fluid and/or a mixture of the propellant fluid and a hemostatic agent (or other agent) to a desired location. As will be described herein, a containment device 50 (e.g., a cartridge or container) may be contained within handle 30 (FIG. 2). A cap 32 may be releasably attached to handle 30 and may control and/or assist in the attachment of containment device 50 to application device 20 within handle 30. Cap 32 may be a proximalmost end of handle 30 in the instance that handle 30 is twisted onto delivery system 10 via threads positioned at a distal end of handle 30 (e.g., threads 30b on handle 30 and threads 26b on application device 20 shown in FIG. 3). An example of delivery system 10 is shown in U.S. Application No. 16/589,633, filed October 1, 2019, the entirety of which is incorporated herein by reference.

With reference to FIGS. 2 and 3, containment device 50 is configured to contain a propellant fluid, such as a gas, e.g., carbon dioxide or any other propellant gas or fluid known in the art. While shown as a cylinder, containment device 50 may be any shape, such as a torpedo-shape, a sphere, or any other shape known in the art for containing gas. For example, containment device 50 could be a carbon dioxide cylinder for insertion into a lumen 34 of handle 30 (FIG. 3). Containment device 50 includes one or more outer walls 50a defining one or more inner chambers 50b, inner chamber(s) 50b configured to contain the propellant fluid. Walls 50a of containment device 50 may be formed of any material suitable for containing the fluid, such as but not limited to a metal alloy, a ceramic, or other material known in the art. The fluid contained in inner chamber 50b of containment device 50 may be under pressure. Accordingly, walls 50a are formed of a material and/or a thickness suitable to contain the fluid at a pressure of, for example, at least approximately 1000 pounds per square inch (PSI), or approximately 850 PSI. For example, gases which may be contained in containment device 50 include carbon dioxide ($CO_2$) having a vapor pressure of approximately 2,000-8,000 kPa at typical device temperatures, or nitrogen ($N_2$) having a vapor pressure less than 40 MPa at typical device temperatures. It will be understood that these gases are examples and are not limiting to the types of gases contained in containment device 50.

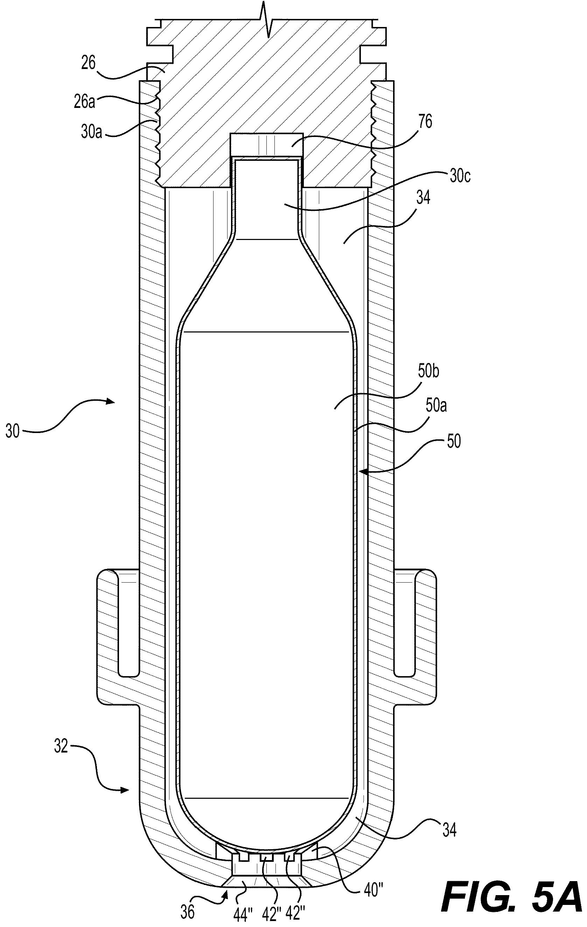

With continued reference to FIG. 3, application device 20 is attached to containment device 50 by inserting containment device 50 into lumen 34 of handle 30. For example, an inlet (e.g., inlet 76 in FIGS. 3 and 5A) of application device 20 may be connected directly to an output, such as a protuberance 30c (FIGS. 3, 4, and 5A) of containment device 50 using a threaded connection, pressure washer adapter, or the like. Protuberance 30c of containment device 50 may extend into inlet 76 of application device 20 and connect, directly or indirectly, to a regulator (not shown). Directly connecting application device 20 to containment device 50 may be suitable for, e.g., a small-volume containment device 20 containing approximately 5 g to 75 g of compressed gas, or preferably approximately 12 g to 40 g of compressed gas, to allow for greater portability of delivery system 10.

Referring to FIG. 1, actuation of one or both of actuating devices 22, 24 of application device 20 causes the fluid to exit delivery system 10 to a target site via tube 100. It will be understood that only one actuating device 22, 24 may need to be actuated in some embodiments. Actuation of one or both actuating devices 22, 24 releases a buildup of pressure within delivery system 10, causing the regulator to release fluid from containment device 50 at a predetermined pressure to delivery system 10 downstream of the regulator. Application device 20 may be, e.g., a garden-hose handle or other pistol-like configuration. Actuating devices 22, 24 may be any push button, trigger mechanism, or other device that, when actuated, opens a valve and releases fluid, as will be described in greater detail herein.

Cap 32 may be attached to a proximal end of handle 30, or handle 30 may be attached to a proximal end of application device 20 (see FIG. 3) such that lumen 34 of handle 30 is fluidly sealed (absent the apertures described herein). Handle 30 may be attached to the proximal end of application device 20 using a threaded connection (e.g., threads 30b and 26b in FIG. 3). For example, after containment device 50 is inserted into lumen 34, handle 30 may be placed at the proximal end of application device 20. An inner surface of a wall 30a at a proximal end of handle 30 may contact containment device 50. Handle 30 may be twisted onto application device 20 via the threaded connection, e.g., threads 30b of handle 30 and threads 26b of application device 20. In some instances, as handle 30 is twisted onto application device 20, the proximal end of wall 30a may urge containment device 50 toward a pierce pin located at inlet 76 of application device 20, such that a distal end 30c of containment device 50 moves into fluid communication with a fluid path of application device 20. Alternatively, containment device 50 may be urged toward the pierce pin by cap 32 when cap 32 is attached to the proximal end of handle 30, as in U.S. Application No. 16/589,633, filed October 1, 2019, the contents of which are incorporated herein by reference in its entirety. In this manner, containment device 50 may be in fluid connection with the fluid path of application device 20, and the fluid propellant may be used to supply an agent from application device 20 to a target site via tube 100. As yet another example, a lever (not shown) may be used to urge containment device 50 in the distal direction to bring containment device 50 into fluid communication with the fluid path of application device 20.

However, sealing containment device 50 within lumen 34 of handle 30 may cause fluid pressure to increase if containment device 50 is not properly connected to application device 20 (e.g., a leak in a seal, eroded threads, etc.) and/or if containment device 50 is damaged and leaks pressurized fluid into lumen 34. Thus, there may be a need to vent fluid from lumen 34 to atmosphere (e.g., outside handle 30).

With reference to FIGS. 2 and 3, apertures 40 are formed in sidewall 30a of handle 30. Apertures 40 fluidly connect an outer surface of handle 30, e.g., an atmosphere or a neutral atmosphere, to lumen 34 of handle 30. Apertures 40 are rectangular in FIG. 2, but are not limited to this shape. For example, apertures 40 may be circular, oval, triangular, curved, spiral, sinusoidal, or irregular. Apertures 40 may extend along an entire length of handle 30 from the proximal end to the distal end, or apertures 40 may be disposed on only a portion of handle 30. Alternatively, apertures 40 may be placed asymmetrically about handle 30 based on ergonomics. For example, apertures 40 may be placed in areas of handle 30 where a user is less likely to grasp handle 30 during use. In this manner, apertures 40 may not be covered by a user's hand during use.

Apertures 40 may have a cross-sectional area sufficient to allow a fluid to pass from lumen 34 to the neutral atmosphere in the event fluid leaks from containment device 50 and does not pass into the fluid pathway of delivery system 10. According to an example, apertures 40 may have a cross-sectional area large enough to permit fluid to escape within approximately 0.5 seconds. For example, once fluid is released into lumen 34 from containment device 50, the fluid may be dispersed from lumen 34 within 0.5 seconds. For example, in the event containment device 50 is not properly attached to the fluid pathway of delivery system 10, or in the event wall 50a of containment device 50 fails and pressurized fluid is released into lumen 34 of handle 30, the pressurized fluid may be vented to atmosphere to prevent pressure buildup in handle 30. In the absence of apertures 40, pressure buildup within lumen 34 of handle 30 may cause wall 30a of handle 30 to burst, which may cause injury to the user and/or the patient.

For example, if the pressure of the fluid in lumen 34 is at room temperature (25 degrees C) at an average pressure of 850 pounds per square inch (PSI), and an opening in containment device 50 is approximately 0.060 inches in diameter, sidewall 30a of handle 30 may rupture if the fluid is not vented within approximately 0.5 seconds. According to an example, a total cross-sectional area of all ventilation openings may be greater than approximately 0.25 square inches. In other words, a sum of the cross-sectional areas of each aperture 40 in handle 30 may be greater than approximately 0.25 square inches. This cross-sectional area of apertures 40 may provide a release of pressurized fluid equal to or less than approximately 0.5 seconds.

As one example, in the event aperture 40 is a single cylinder (extending through sidewall 30a from lumen 34 to atmosphere) having a circular cross-section, a minimum radius of the circular cross-section of aperture 40 may be determined based on Formula 1. In Formula 1, R is the radius of the circular cross-section of aperture 40, Q is the flow rate of the fluid through aperture 40 in standard liters per minute (Q is predetermined value based at least in part on the time in which lumen 34 is to be vented, e.g., 0.5 seconds), η is the viscosity of the fluid flowing through aperture 40, L is a thickness of handle 30 (e.g., a length of aperture 40 from lumen 34 to the atmosphere), and ΔP is a pressure difference (in PSI) between a pressure inside lumen 34 and atmospheric pressure..

Formula 1:

$$R \geq \sqrt[4]{\frac{8Q\eta L}{\Delta P \Pi}}$$

In some examples, a membrane (not shown) may cover each aperture 40, or a burst disc or pressure relief valve may be used in place of one or more apertures 40. The membrane may be provided on an outer surface and/or an inner surface of handle 30, may be any color, and may be translucent or transparent. A material of the membrane may include preformed separation zones and/or or perforations to assist in rupturing at a threshold. The membrane may be made of a flexible material and/or a material suitable to rupture when a pressure within lumen 34 exceeds a threshold, e.g., greater than or equal to approximately 14 PSI. In some examples, the pressure may change based on atmospheric pressure outside handle 30 (e.g., atmospheric pressure may change based on a location of use of the device). Similarly, a burst disc or pressure relief valve may open once the pressure with lumen 34 exceeds a threshold.

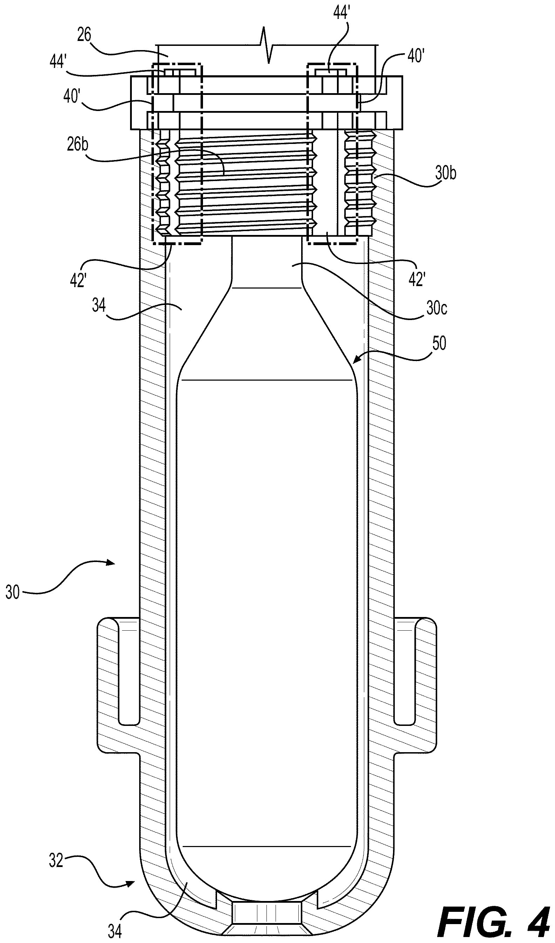

Apertures 40' according to another example are shown in FIG. 4. As discussed herein, containment device 50 may be attached to application device 20 via threads 30b on handle 30 and threads 26b on application device 20. One or more apertures 40' may be formed through and 26b. For example, apertures 40' may extend from an opening 42' at a proximalmost end of threads 26b to an opening 44' at a distalmost end of threads 26b. Opening 44' is open to and/or in fluid communication with the external atmosphere. In some instances, apertures 40' may be formed by removing a portion of threads 26b at locations around a circumference of the threaded portion of application device 20. That portion may be part of a regulator or pierce system. For example, threads 26b may be disconnected at a same circumferential location along an entire length of threads 26b to form apertures 40', as shown in FIG. 4. Alternatively, or additionally, apertures 40' may be formed in threads 30b of handle 30. In some instances, apertures 40' formed on handle 30 may match up with apertures 40' on application device 20 when handle 30 is completely attached to application device 20. This may increase the cross-sectional area of apertures 40', which may increase the ventilation of pressurized fluid from lumen 34. In some cases, a membrane may cover one or both of openings 42', 44' of apertures 40'. The membrane may be made of a flexible material and/or a material suitable to rupture when a pressure within lumen 34 exceeds a threshold, such as the pressures mentioned above.

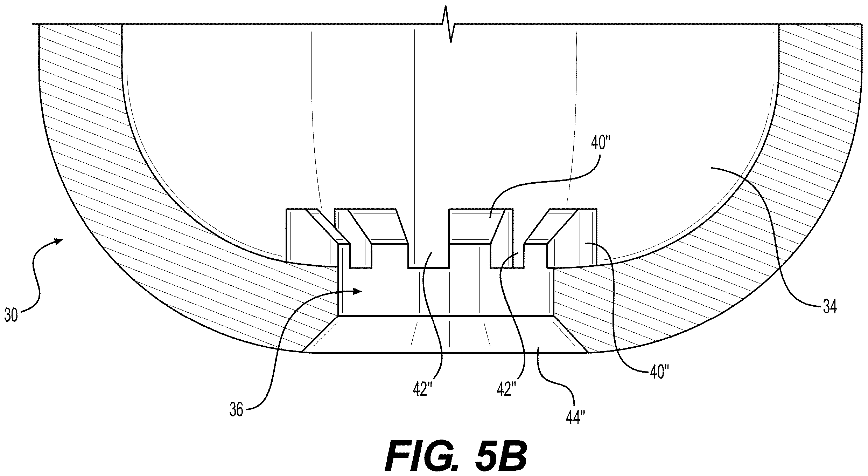

Another example of a fluid release mechanism is shown with reference to FIGS. 5A and 5B. Handle 30 includes a member 36 at the proximalmost end of handle 30. Member 36 includes crenellations 40" (supports), and a plurality of apertures 42" defined between adjacent crenellations 40". In some instances, a portion or entirety of member 36 may include a material that increases a friction coefficient between member 36 and containment device 50 to assist in urging containment device 50 in the distal direction. As discussed herein, containment device 50 may be urged toward the pierce pin by an inner surface of handle 30 (e.g., cap 32 or the proximal end of handle 30) as handle 30 is twisted onto application device 20, or as cap 32 is twisted onto application device 20. In this situation, crenellations 40" may contact a proximalmost outer surface of containment device 50 and support device 50 above apertures 42". As handle 30 and/or cap 32 are moved toward application device 20 via a twisting motion, crenellations 40" may contact the proximalmost outer surface of containment device 50 to aid in the movement of containment device 50 in the distal direction (e.g., in the direction indicated by arrow B in FIG. 1). Crenellations 40" may assist in moving containment device in the distal direction, and/or may aid in creating a space between an outermost surface of containment device 50 and an innermost surface of sidewall 30a. This space (e.g., a portion of lumen 34) may allow any fluid escaping from containment device 50 to pass unimpeded to apertures 42" and to an outside atmosphere, as discussed herein. It will be understood that any number of crenellations 42" may be formed, for example, two, three, or more crenellations 42".

A proximal-facing opening 44" may be formed in member 36. Opening 44" is fluidly connected to each of apertures 42", which are in turn fluidly connected to lumen 34 of handle 30. When handle 30 is connected to application device 20, apertures 42" and opening 44" allow any pressurized fluid buildup within lumen 34 to be vented to an atmosphere. In an example, venting may occur in approximately 0.5 seconds. For example, as discussed herein, an opening or an aperture may have a size of approximately 0.25 square inches or greater to allow any fluid buildup to pass to the outside atmosphere. In this case, the sum of the cross-sectional areas of all apertures 42" may be greater than or equal to 0.25 square inches, and the cross-sectional area of opening 44" may be greater than or equal to approximately 0.25 square inches. It will also be understood that any pathway formed between apertures 42" and opening 44" (e.g., a sidewall of member 36) may have a cross-sectional area of greater than or equal to approximately 0.25 square inches. It will be understood that member 36 may be integral with handle 30, and not a separate part or component. For example, crenellations 40" may be formed integrally with handle 30, e.g., integrally molded, leaving apertures 42" and opening 44" in the molding process.

While the apertures described herein have been described as having a total cross-sectional area of greater than or equal to approximately 0.25 square inches, it will be understood that this is an example and may change based on design factors. For example, pressurized fluid may be dispersed from lumen 34 in less than or equal to approximately 0.5 seconds. If the fluid is not dispersed from lumen 34 in this timeframe or other suitable timeframe depending on design factors (including the strength of sidewall 30a), the structural integrity of sidewall 30a may fail, causing injury. In some instances, containment device 50 may include a pressurized fluid greater than 850 PSI, and/or delivery system 10 may be designed for use at a temperature different from room temperature (e.g., approximately 25 degrees C). In these instances, the apertures and openings described herein may be designed to have a cross-sectional area large enough to disperse the pressurized fluid in less than or equal to approximately 0.5 seconds, or other suitable timeframe, based on Formula 1 and the corresponding discussion above. It will also be understood that one or more of apertures 40, 40', and/or 42" may be combined in a same delivery system 10 to provide fluid dispersement.

It will be apparent to those skilled in the art that various modifications and variations can be made to the disclosed device without departing from the scope of the disclosure. Other embodiments of the disclosure will be apparent to those skilled in the art from consideration of the specification and practice of the invention disclosed herein. It is intended that the specification and examples be considered as exemplary only, with a true scope and spirit of the invention being indicated by the following claims.

The following aspects are preferred embodiments of the invention. 1. A device configured to deliver a pressurized fluid, the device comprising:

a body having an input opening for receiving the pressurized fluid and an output opening for delivering the pressurized fluid;
a handle defining a lumen, wherein the lumen is configured to receive a container containing the pressurized fluid; and
at least one aperture fluidly connecting the lumen to an atmosphere external of the device.

2. The device according to aspect 1, wherein the at least one aperture is defined by a wall of the handle.

3. The device according to any of the preceding aspects, wherein the at least one aperture includes a plurality of apertures, and wherein the plurality of apertures are arranged in an asymmetrical pattern on the handle.

4. The device according to aspect 1, wherein a distal end of the handle is configured to be connected to the body via screw threads, and wherein the at least one aperture extends through a portion of the screw threads.

5. The device according to aspect 4, wherein the handle includes one or more screw threads, wherein the body includes one or more screw threads, wherein the one or more apertures are configured to be formed through the one or more screw threads of each of the handle and the body, and wherein the one or more apertures on the handle and the one or more apertures on the body are configured to align when the handle is attached to the body.

6. The device according to aspects 1 or 2, wherein the one or more apertures is a single aperture disposed at a proximalmost end of the handle, and wherein the single aperture faces a proximal direction.

7. The device according to aspect 6, wherein the device includes a plurality of crenellations and a plurality of openings defined between adjacent crenellations, and wherein the openings are fluidly connected to the single aperture.

8. The device according to aspect 7, wherein the plurality of crenellations are configured to contact a proximalmost end of the container when the container is attached to the device.

9. The device according to aspects 7 or 8, wherein a sum of a cross-sectional area of each of the plurality of openings is equal to a cross-sectional area of the single aperture.

10. The device according to any of the preceding aspects, wherein only proximally facing surfaces of an outermost surface of the container contacts an innermost surface of the handle when the container is connected to the device.

11. The device according to any of the preceding aspects, wherein the at least one aperture is configured to release the pressurized gas in a time equal to or less than 0.5 seconds.

12. The device according to any of the preceding aspects, wherein the body is configured to form a hole in the container, wherein the pressurized fluid is configured to pass from the container via the hole, and wherein a diameter of the hole is less than or equal to 0.060 inches.

13. The device according to aspects 1-8 and 10-12, wherein a cross-sectional area of a sum of each of the at least one aperture is equal to or greater than approximately 0.25 square inches.

14. The device according to any of the preceding aspects, further comprising:

the container; and
a membrane covering each aperture of the at least one aperture, wherein the membrane is configured to rupture when a pressure of the pressurized fluid within the lumen exceeds a threshold.

15. The device according to any of the preceding aspects, wherein the pressurized fluid has a pressure of approximately 14 pounds per square inch.

**Claims**

1. A medical device (20) configured to deliver an agent, the medical device comprising:

a body;
a handle (30) defining a lumen (34), wherein the lumen (34) is configured to receive a container (50) containing the pressurized fluid;
a member (36) disposed at a proximal end of the handle (30), wherein the member (36) is configured to urge the container (50) within the handle (30) towards a distal direction into fluid communication with the body;
an inlet (76) disposed at a distal end of the handle (30), wherein the inlet (76) includes a pierce pin configured to release the pressurized fluid from the container (50) in response to the member (36) urging the container (50) into the pierce pin.

2. The medical device of claim 1, further including a cap (32) disposed at the proximal end of the handle (30), wherein the cap (32) is configured to attach the container (50) within the handle (30) to the body.

3. The medical device of claim 2, wherein the cap (32) includes a plurality of threads (30b), wherein the plurality of threads (30b) are configured to mesh with a plurality of threads (26b) on the body to secure the handle (30) onto the body.

4. The medical device of claim 3, wherein the cap (32) is configured to urge the container (50) towards the pierce pin at the distal end of the handle (30) when the handle (30) is secured to the body via the plurality of threads (30b) on the handle (30) meshing with the plurality of threads (26b) on the body.

5. The medical device of claim 4, wherein the handle (30) is configured to rotate relative to the body to move the container (50) towards the pierce pin, thereby establishing fluid communication between the container (50) and a fluid path within the body.

6. The medical device of any one of the preceding claims, wherein the member (36) includes an opening (44"), at least one support (40"), and at least one aperture (42").

7. The medical device of claim 6, wherein the at least one support (40") includes a plurality of supports (40") that are configured to move the container (50) towards the distal end of the handle (30).

8. The medical device of claim 7, wherein the plurality of supports (40") are configured to create space between an outermost surface of the container (50) and an innermost surface of the handle (30).

9. The medical device of claim 6, wherein the opening (44") is configured to be proximal facing such that the pressurized fluid received within the lumen (34) is vented out of the handle (30) at the proximal end via the opening (44") on the member (36").

10. The medical device of claim 6, wherein the at least one aperture (42") includes a plurality of apertures (42") that are in fluid communication with the lumen (34).

11. The medical device of claim 5, wherein the plurality of apertures (42") are defined between a first support (40") and a second support (40") of the at least one support (40").

12. The medical device of claim 5, wherein the opening (44") is in fluid communication with the lumen (34) via each of the plurality of apertures (42"), wherein the opening (44") is configured to release a buildup of the pressurized fluid within the lumen (34) into an atmosphere via the plurality of apertures (42").

13. The medical device of any one of the preceding claims, further including a lever configured to urge the container (50) in the distal direction towards the pierce pin to release the pressurized fluid from the container (50) in response to actuation of the lever.

14. The medical device of any one of the preceding claims, wherein the container (50) is configured to release the pressurized fluid into the body to mix with and deliver the agent out of the medical device.

15. The medical device of any one of the preceding claims, the body including an output opening for delivering the pressurized fluid.

**FIG. 1**

**FIG. 2**

FIG. 3

FIG. 4

**FIG. 5A**

**FIG. 5B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63081659
- US 58963319